# EUROPEAN PATENT APPLICATION

(11) **EP 4 644 125 A2**
(43) Date of publication of application: **05.11.2025**
(21) Application number: 25196319.5
(22) Date of filing: 22.03.2019
(51) Int. Cl.: B33Y 70/00

(54) **3D PRINTING COMPOSITION FOR BIOMATERIALS**

(30) Priority: 22.03.2018 EP 18163397
(62) Divisional of application: 19711613.0
(71) Applicant: Tissium SA, 75012 Paris (FR)
(72) Inventor: COLLIN, Estelle, 94140 Alfortville (FR); PEREIRA, Maria, 2400-441 Parceiros (PT); MAIA E SILVA, João Reina, 4150-175 Porto (PT); CIRILLO, Valentina, 92200 Neuilly sur Seine (FR); LAMAZOUADE, Julien, 33460 Arsac (FR); KEAVENEY, Shane, Sligo (IE); O'CEARBHAILL, Eoin D., Dublin (IE)
(74) Representative: Finnegan Europe LLP

(57) **Abstract**

A 3D printing resin composition for a biomaterial and method for 3D printing the composition, wherein the composition comprises:
(i) a pre-polymer comprising a polymeric unit of the general formula (-A-B-)ₙ, wherein A represents a substituted or un-substituted ester, B represents a substituted or un-substituted acid ester comprising at least two acid ester functionalities, and n represents an integer greater than 1,
(ii) at least one photo-initiator, and
(iii) at least one light blocker.

## Description

### FIELD OF INVENTION

The present invention relates to a 3D printing composition for a biomaterial, more specifically a 3D printing composition for a biocompatible implant, a method of printing (e.g. a biocompatible implant) using the composition, a biomaterial obtainable therefrom, use of the biomaterial and method of using the biomaterial.

### BACKGROUND OF THE INVENTION

Over the past fifty years, biomaterials have been widely used to replace and/or restore the function of traumatized or degenerated tissues or organs, and various forms of implants or other medical devices have been developed based on them.

Three-dimensional (3D) printing, also known as additive manufacturing, can be utilised in the production of implants and other medical devices. Implants and medical devices include implantable structures such as scaffolds, stents, constructive and supportive components. One advantage of 3D printing is the flexibility in defining parameters in order to produce a custom made structure which may be personalised for the end user. Another advantage is the ability to produce complex structures at high resolution. This enables the production of structures with microstructural parameters from computer generated designs, which are particularly useful in the field of biomaterials, especially in the field of implants or other medical devices.

According to known methods of 3D printing construction of a three-dimensional structure is typically performed in a step-wise manner, layer by layer and includes for example extrusion, direct energy deposition, solidification of powder, photopolymerisation and sheet lamination. In particular, layer formation is performed through solidification of photo curable resin under the action of visible or UV light irradiation. Alternatively, the three- dimensional structure can be created continuously from a liquid interface (see for example WO2014126837 or US7892474). All these 3D printing methods rely on the properties of the 3D printing composition to define the microstructural parameters and possibly biochemical properties of the printed structure. Hence, the properties of a 3D printed structure are limited not only by the capability of the printing method, but also by the printing composition used.

Compositions currently utilised in 3D printing of implantable structures include biodegradable polymeric materials such as polylactic acid (PLA), poly-1-lysine (PLL), poly(lactic-co-glycolic acid) (PLGA) and poly-ε-caprolactone (PLC). While these polymers have advantageous properties, limitations still remain in the achievable resolution of the structures produced by 3D printing these materials. Moreover, many of these compositions are not photocurable and therefore can only be printed using limited number of 3D printing techniques and thus cannot be used with Stereo Lithography Apparatus (SLA) or Digital Light Processing (DLP).

High resolution is particularly desirable for a biomaterial, especially for an implant or other medical device, as it enables the enhancement of its properties such as elasticity, flexibility or porosity and limits its impact on cellular response. Increasingly smaller structures are also possible with higher resolution, which is an important requirement of an implantable structure. The printing resolution achieved is normally dependent on the equipment used and the physico-chemical characteristic of the resin used.

An ideal biomaterial should be relatively inert, able to withstand mechanical impact and contortion, and biodegradable. Biodegradability is especially relevant in repairing damaged tissue or replacing/implanting tissue whereby the presence of the structure inside the body is intended to be transient. The repair of nervous tissue is one example. Where a nerve has been severed or damaged, a biocompatible nerve conduit can be introduced which provides a guide for the nerve to regrow (e.g. Anderson et al., 2015, Crit. Rev. Biomed. Eng., 43, 131-159). Nerve conduits may also contain a nerve graft used to replace any damaged or missing nervous tissue, therefore the ability to harbour and retain tissue, cells, supporting growth factors and/or pharmaceutical compositions is highly desirable. Accordingly, the ability to specify and control both the microstructure and nanostructure, and possibly composition, of a biocompatible implant such as a nerve conduit is increasingly required.

Methods of 3D printing biocompatible implants with a high resolution have been explored. WO 2016176444 describes methods of 3D printing biomedical devices using photo-curable polymer-based inks including known biodegradable polymers in the presence of a UV-absorber. This publication focuses on the need to be able to rapidly fabricate microstructures such as stents, with high fidelity. However, this approach does not address the disadvantages of using these types of polymers including difficulty in achieving accuracy at microstructure level and post-processing product faults.

Yeh, et al., 2016, Biofabrication (8), 1-10, describes using acrylated polyglycerol sebacate (Acr-PGS) to generate scaffolds with increased elastic properties. This describes synthesizing and blending two Acr-PGS macromers. Macromers with too high viscosity resulted in fragile products which cracked, and those with too low a viscosity resulted in a loss of structural resolution.

Therefore, there still exists a need for an improved and commercially viable 3D printing polymer-based composition which is capable of producing a high resolution structure whilst retaining the mechanical tensile strength and stability required for a biomaterial, in particular a biocompatible and biodegradable implant.

Furthermore, materials that degrade through surface erosion and that present minimal swelling, are particularly needed and attractive for applications where thin structures should be mechanically stable and a high resolution should be maintained upon implantation.

### SUMMARY OF THE INVENTION

The present invention provides 3D printing resin composition for a biomaterial, wherein the composition comprises:
(i) a pre-polymer comprising a polymeric unit of the general formula (-A-B-)ₙ, wherein A represents a substituted or un-substituted ester, B represents a substituted or unsubstituted acid ester comprising at least two acid ester functionalities, and n represents an integer greater than 1,
(ii) at least one photo-initiator, and
(iii) at least one light blocker.

The present invention also provides a method of 3D printing a biomaterial, wherein the method comprises:
(a) 3D printing the resin composition of the present invention, and
(b) washing the 3D printed composition with a solvent

After washing, additional steps may be taken to post-cure the samples using, for example, temperature or light radiation.

Step (a) of the 3D printing method can include the steps of :
(i) delivering a layer of the resin composition of the invention according to printing parameters,
(ii) exposing the layer of the resin composition of the invention to light to cure the polymer of the resin and produce a solidified resin layer and
(iii) repeating step (i) and (ii) with each successive layer built upon the previous layer to obtain 3D printed composition.

The present invention also provides a biomaterial, preferably a biocompatible implant obtainable by the method of the present invention.

The present invention also provides a method of repairing or supporting tissue. According to one embodiment, this relates to nervous tissue, the method comprising applying the biomaterial of the present invention, preferably a biocompatible implant, to the nervous tissue. According to another embodiment, this relates to soft tissues (including for example breast tissue and skin), the method comprising applying the biomaterial of the present invention, preferably a biocompatible implant, to soft tissues. According to a further embodiment, this relates to bone tissue, the method comprising applying the biomaterial of the present invention, preferably a biocompatible implant, to the bone tissue.

The present invention further provides a method for producing biomaterial, preferably a biocompatible implant obtainable by the method of the present invention, for different uses or functions. This may include, for example, stents, filters, valves, membranes (deployable or not), drug delivery vehicles (such as microneedles and capsules), drains, etc. Furthermore, it may also be applicable for *in vitro* applications, such as biocompatible resin/material for, for example, cellular assays, lab on a chip or other organ on a chip devices.

### BRIEF DESCRIPTION OF THE FIGURES

Figure 1 shows the viscosity analysis of 3D printing resin compositions at different temperatures according to the present invention.
Figure 2 shows the viscosity analysis of 3D printing resin compositions in the presence of various solvents according to the present invention.
Figure 3 shows the elastomeric properties of a 3D printed resin composition with and without solvent according to the present invention.
Figure 4 shows the extent of shrinkage of a 3D printed resin composition with and without solvent according to the present invention.
Figure 5 shows cross-sectional views of 3D printed resin nerve conduits according to the present invention.
Figure 6 shows (A) SEM image of a conduit printed in Asiga hardware with PGSA composition not supplemented by any solvent and (B) CAD image sent to the printer for fabrication of the object. (1) and (2) represent the points of measurement.
Figure 7 (A) shows temperature profile of the resin during printing. (B) shows SEM images of 3D printed wrap at low temperature.
Figure 8 shows geometry of the 3D construct with a 1mm base and detailed features on top in presence or absence of BBOT.
Figure 9 shows A: nerve wrap 3D printed according to the invention; B: the wrap can be easily opened with surgical tweezers; C: it recovers its original shape when tweezers are removed.

### DETAILED DESCRIPTION OF THE INVENTION

### 3D Printing Composition

The 3D printing resin composition for a biomaterial according to the present invention comprises:
(i) a pre-polymer comprising a polymeric unit of the general formula (-A-B-)ₙ, wherein A represents a substituted or un-substituted ester, B represents a substituted or unsubstituted acid ester comprising at least two acid ester functionalities, and n represents an integer greater than 1,
(ii) at least one photo-initiator, and
(iii) at least one light blocker.

The term "pre-polymer" refers to linear or branched polymers or monomers that have the capacity to be further polymerised or crosslinked under appropriate conditions.

The pre-polymer of the composition according to the present invention can be manufactured in a number of ways including as outlined in WO 2016/202984 A1.

### Pre-polymer

The pre-polymer according to the present invention comprises a polymeric unit of the general formula (-A-B-)ₙ, wherein A represents a substituted or un-substituted ester, B represents a substituted or un-substituted acid or acid ester comprising at least two acid or acid ester functionalities; and n represents an integer greater than 1.

Component A may be derived from a polyol, such as a diol, triol, tetraol or greater, or any mixture thereof. Suitable polyols include diols, such as alkane diols; triols, such as glycerol, trimethylolpropane, triethanolamine; tetraols, such as erythritol, pentaerythritol; and higher polyols, such as sorbitol. Unsaturated diols, such as tetradeca-2,12-diene-1,14-diol, or other diols including macromonomer diols such as, for example polyethylene oxide, and N-methyldiethanoamine (MDEA) can also be used. Preferably, the polyol is substituted or unsubstituted glycerol.

Component B may be derived from a polyacid, such as a diacid or higher order acid, or any mixture thereof. A wide variety of diacid, or higher order acids, can be used. Exemplary acids include, but are not limited to, glutaric acid (5 carbons), adipic acid (6 carbons), pimelic acid (7 carbons), sebacic acid (8 carbons), and azelaic acid (nine carbons). Exemplary long chain diacids include diacids having more than 10, more than 15, more than 20, and more than 25 carbon atoms. Non-aliphatic diacids can also be used. For example, versions of the above diacids having one or more double bonds can be used to produce polyol-diacid co-polymers. Preferably the diacid is substituted or unsubstituted sebacic acid.

Polyol-based polymers described in US Patent Application Publication 2011-0008277, U.S. Pat No 7,722,894 and U.S. Pat No 8,143,042, the contents of which are hereby incorporated by reference, can also be used as a pre-polymer to form elastomeric polymeric materials

Several substituents, such as amines, aldehydes, hydrazides, acrylates and aromatic groups, alcohols, carboxylic acids, can be incorporated into the carbon chain, and/or on Component A and/or on Component B. Exemplary aromatic diacids include terephthalic acid and carboxyphenoxy-propane. The diacids can also include substituents as well. For example, reactive groups like amine and hydroxyl can be used to increase the number of sites available for crosslinking. Amino acids and other biomolecules can be used to modify the biological properties. Aromatic groups, aliphatic groups, and halogen atoms can be used to modify the inter-chain interactions within the polymer.

The pre-polymer may further comprise a polyamide or polyurethane backbone. For example, polyamine (comprising two or more amino groups) may be used to react with polyacid together with polyol or after reacting with polyol. Exemplary poly(ester amide) includes those described in Cheng, et al., Adv. Mater. 2011, 23, 1195-11100, the contents of which are herein incorporated by reference. In other examples, polyisocianates (comprising two or more isocyanate groups) may be used to react with polyacid together with polyol or after reacting with polyol. Exemplary polyester urethanes include those described in US2013231412.

The weight average molecular weight of the pre-polymer, measured by Gel Permeation Chromatography equipped with a refractive index, may be from about 1,000 Daltons to about 1,000,000 Daltons, from about 1,000 Daltons to about 1,000,000 Daltons, preferably from about 2,000 Daltons to about 500,000 Daltons, more preferably from about 2,000 Daltons to about 250,000 Daltons, most preferably from about 2,000 Daltons to about 100,000 Daltons. The weight average molecular weight may be less than about 100,000 Dalton, less than about 75,000 Daltons, less than about 50,000 Daltons, less than about 40,000 Daltons, less than about 30,000 Daltons, or less than about 20,000 Daltons. The weight average molecular weight may be from about 1000 Daltons to about 10,000 Daltons, from about 2000 Daltons to about 10,000 Daltons, from about 3000 Daltons to about 10,000 Daltons from about 5,000 Daltons to about 10,000 Daltons. Preferably, it is about 3000 Daltons.

The term "about" as used herein means within 10%, preferably within 8%, and more preferably within 5% of a given value or range. According to specific embodiment, "about X" means X.

The pre-polymer may have a polydispersity, measured by Gel Permeation Chromatography equipped with a refractive index, below 20.0, more preferably below 10.0, more preferably below 5.0, and even more preferably below 2.5. Preferably, it is about 2.5.

The pre-polymer may have a melt viscosity at 80°C between 100 and 2000 cP, more preferably between 200 and 1000 cP and even more preferably between 300 and 500 cP.

The pre-polymer may have an acid number between 1 and 200 mg KOH/g of polymer, more preferably between 10 and 100 mg KOH/g of polymer, and even more preferably between 50 and 100 mg KOH/g of polymer. Preferably, it is about 80 mg KOH/g of polymer

The molar ratios of the polyol to the polyacid in the pre-polymer may be 1:1, 1:2, 1:3, 1:4, 1:5, 1:6,1:7,1:8,1:9 and 1:10. 10:1, 9:1, 8:1, 7:1, 6:1, 5:1, 4:1, 3:1, 2:1, 1:1. The molar ratios of polyol to the polyacid may also be 2:3, 3:2, 3:4, or 4:3. The polymer may also be the result of a mixture of two or more different ratios. Preferably, it is about 1:1.

### Activated Pre-polymer

The pre-polymer of the present invention is preferably activated. It can be activated by introducing functional groups that can react or be reacted to form crosslinks. The pre-polymer is activated by reacting one or more functional groups on the pre-polymer backbone with one or more functional groups that can react or be reacted to form crosslinks resulting in cured polymer.

Suitable functional groups to be activated on the pre-polymer backbone include hydroxy groups, carboxylic acid groups, amines, and combinations thereof, preferably hydroxy and/or carboxylic acid. The free hydroxyl or carboxylic acid groups on the pre-polymer can be activated by functionalizing the hydroxy groups with a moiety which can form a crosslink between polymer chains. The groups that are activated can be free hydroxyl or carboxylic acid groups on A and/or B moieties in the pre-polymer.

The free hydroxy or carboxylic groups can be functionalized with a variety of functional groups, for example vinyl groups. Vinyl groups can be introduced by a variety of techniques known in the art, such as by vinylation or acrylation. According to the present invention, vinyl groups contain the following structure -CR₁=CR₂R₃ wherein R₁, R₂, R₃ are independently from one another, selected in the group consisting of H, alkyl such as methyl, ethyl, aryl such as phenyl, substituted alkyl, substituted aryl, carboxylic acid, ester, amide, amine, urethane, ether, and carbonyl.

Preferably, the functional group is or contains an acrylate group. According to the present invention, acrylate groups are moieties containing substituted or unsubstituted acryloyl group. The acrylate may contain the following group: -C(=O)-CR₁=CR₂R₃, wherein R₁, R₂, R₃ are independently from one another, selected in the group consisting of H, alkyl such as methyl or ethyl, aryl such as phenyl, substituted alkyl, substituted aryl, carboxylic acid, ester , amide, amine, urethane , ether, and carbonyl.

Preferably, R₁, R₂ and R₃ are H; or R₁ is CH₃, R₂ and R₃ are H; or R₁ and R₂ are H and R₃ is CH₃; or R₁ and R₂ are H and R₃ is phenyl.

Vinyl groups can also be incorporated in the backbone of the pre-polymer using free carboxyl groups on the pre-polymer. For example, hydroxyethyl methacrylate can be incorporated through the COOH groups of the pre-polymer using carbonyl diimidazole activation chemistry.

The degree of activation can vary and can be from 0.2 to 0.9 mol/mol of polyacid or polyol, preferably from 0.3 to 0.8 mol/mol of polyacid or polyol and most preferably from 0.4 to 0.6 mol/mol of polyacid or polyol, such as 0.5 mol/mol of polyacid or polyol for achieving optimal bust performance properties at room temperature or elevated temperature up to 40 °C, preferably 37 °C. It is most preferred when the degree of activation is as described above and the reactive functional group is acrylate i.e. degree of acrylation as above.

The activated pre-polymer preferably has the general formula (I): 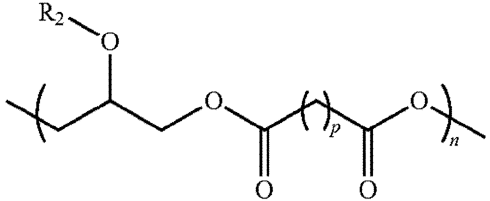 wherein n and p each independently represent an integer equal or greater than 1, and wherein R₂ in each individual unit represents hydrogen or a polymer chain or -C(=O)-CR₃=CR₄R₅, wherein R₃, R₄, R₋₅ are independently from one another, selected in the group consisting of H, alkyl such as methyl or ethyl, aryl such as phenyl, substituted alkyl, substituted aryl, carboxylic acid, ester , amide, amine, urethane , ether, and carbonyl.

Preferably, R₃, R₄ and R₅ are H; or R₃ is CH₃, R₄ and R₅ are H; or R₃ and R₄ are H and R₅ is CH₃; or R₃ and R₄ are H and R₅ is phenyl.

Preferably, p is an integer from 1-20, more preferably from 2-10, even more preferably from 4-10. It is most preferred when p=8.

The preferred pre-polymer has the following structure: 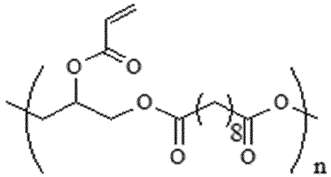 wherein n represents an integer equal or greater than 1

In addition to acrylates or other vinyl groups, other agents can be used to activate the pre-polymer. Examples of such agents include, but are not limited to, glycidyl, epichlorohydrin, triphenylphosphine, diethyl azodicarboxylate (DEAD), diazirine, divinyladipate, and divinylsebacate with the use of enzymes as catalysts, phosgene-type reagents, di-acid chlorides, bis-anhydrides, bis-halides, metal surfaces, and combinations thereof. Agents may further include isocyanate, aldehyde, epoxy, vinyl ether, thiol, DOPA residues or N-Hydroxysuccinimide functional groups.

The activated pre-polymer can be further reacted with one or more additional materials to modify the crosslinks between the polymer chains. For example, prior to or during curing/crosslinking, one or more hydrogel or other oligomeric or monomeric or polymeric precursors (e.g., precursors that may be modified to contain acrylate groups) such as poly(ethylene glycol), dextran, chitosan, hyaluronic acid, alginate, other acrylate based precursors including, for example, acrylic acid, butyl acrylate, 2-ethylhexyl acrylate, methyl acrylate, ethyl acrylate, acrylonitrile, n-butanol, methyl methacrylate, acrylic anhydride, methacrylic anhydride and TMPTA, trimethylol propane trimethacrylate, pentaerythritol trimethacrylate, pentaerythritol tetramethacrylate, ethylene glycol dimethacrylate. dipentaerythritol penta acrylate, Bis-GMA (Bis phenol A glycidal methacrylate) and TEGDMA (tri-ethylene, glycol dimethacrylate), sucrose acrylate; other thiol based precursors (monomeric or polymeric); other epoxy based precursors; and combinations thereof, can be reacted with the acrylated pre-polymer (e.g., PGSA).

The activated pre-polymer may be manufactured in the presence and/or mixed with a coloring agent. Preferred examples of coloring agents are the ones recommended by the FDA for use in medical devices, pharmaceutical products or cosmetics. See http://www.fda.gov/ForIndustry/ColorAdditives/ColorAdditiveInventories/. More preferably, this agent is FD&C 1.

Preferably according to the present invention, it is desirable to control presence of anhydrides in the composition. Preferably according to the present invention the molar ratio of the total content of grafted anhydride in the composition is less than 0.05mol/mol of polyacid as measured by nuclear magnetic resonance (NMR). Preferably there is no grafted anhydride present in the composition. More preferably there is no anhydride, grafted or non-grafted, present in the composition.

The content of grafted anhydride in the composition can be controlled during synthesis by ethanol capping or using any other nucleophilic substitution reaction. These chemical reactions are well known in the art. Suitable reagents for this reaction include alcohols, amines or sulfhydryl compounds. The addition of ethanol is preferably at a temperature in the range of 30 to 50 °C, preferably 35 to 45°C, for example 40 °C. The duration of the ethanol capping step is conducted preferably during 10 and 40 hours, more preferably during 24 hours. The volumetric ratio of polymer solution (~10% w/v) to ethanol is in the range of 20:1, more preferably in the range of 10:1 and even more preferably in the rage of 5:1.

A method for manufacturing the activated pre-polymer of the present invention comprises:
i) polycondensation of a first component comprising two or more functionalities of the general formula -OR, where R of each group is independently hydrogen or alkyl; and a second component comprising two or more acid ester functionalities;
ii) activation of the pre-polymer made by step i);
iii) control of anhydride content; optionally
iv) blocking free hydroxyl groups; and/or optionally
v) purification of the activated pre-polymer made by steps ii) and/or iii) and/or iv).

The said first component may be a polyol or a mixture of polyols, such as a diol, triol, tetraol or greater. Suitable polyols include diols, such as alkane diols; triols, such as glycerol, trimethylolpropane, triethanolamine; tetraols, such as erythritol, pentaerythritol; and higher polyols, such as sorbitol. Unsaturated diols, such as tetradeca-2,12-diene-1,14-diol, or other diols including macromonomer diols such as polyethylene oxide, and N-methyldiethanoamine (MDEA) can also be used. Preferably, the polyol is substituted or unsubstituted glycerol.

The said second component may be a polyacid, such as a diacid or higher order acid or a mixture of diacids and/or polyacids. A wide variety of diacid, or higher order acids, can be used. Exemplary acids include, but are not limited to, glutaric acid (5 carbons), adipic acid (6 carbons), pimelic acid (7 carbons), sebacic acid (8 carbons), and azelaic acid (nine carbons). Exemplary long chain diacids include diacids having more than 10, more than 15, more than 20, and more than 25 carbon atoms. Non-aliphatic diacids can also be used. For example, versions of the above diacids having one or more double bonds can be used to produce polyol-diacid co-polymers.

Exemplary aromatic diacids include terephthalic acid and carboxyphenoxy-propane. The diacids can also include substituents as well, for example amine and hydroxyl substituents.

Preferably the diacid is substituted or unsubstituted sebacic acid.

The said first and second component are added together in a first component: second component molar ratio range of 0.5:1 to 1.5:1, preferably 0.9:1.1 and most preferred 1:1. Where the first component is glycerol and the second component is sebacic acid and added in a 1:1 molar ratio, there are three hydroxyl groups on glycerol for two carboxyl groups on the sebacic acid. Therefore the extra hydroxyl group on glycerol is used during the activation step.

The conditions for step i) are not especially limited but may include a temperature range of 100 to 140°C, preferably 120 to 130°C, an inert atmosphere, preferably comprising nitrogen, and under vacuum.

The activating agent of step ii) is preferably an acrylating agent which comprises an acrylate group which are moieties containing substituted or unsustituted acryloyl group. The acrylate may contain the following group: -C(=O)-CR₁=CR₂R₃, wherein R₁, R₂, R₃ are independently from one another, selected in the group consisting of H, alkyl such as methyl or ethyl), aryl such as phenyl, substituted alkyl, substituted aryl, carboxylic acid, ester , amide, amine, urethane , ether, and carbonyl.

Preferably, R₁, R₂ and R₃ are H; or R₁ is CH₃, R₂ and R₃ are H; or R₁ and R₂ are H and R₃ is CH₃; or R₁ and R₂ are H and R₃ is phenyl.

Most preferably, the acrylating agent is acryloyl chloride.

During the acrylation process, anhydrides can be formed resulting from the reaction of the acrylated monomer with any carboxylic acid groups. According to preferred embodiment, the anhydride content is controlled in step (iii) by ethanol capping or using any other nucleophilic substitution reaction. Suitable reagents for this step (iii) include alcohols, amines or sulfhydryl compounds. The addition of ethanol is preferably at a temperature in the range of 30 to 50 °C, preferably 35 to 45°C, for example 40 °C. The duration of the ethanol capping step is conducted preferably during 10 and 40 hours, more preferably during 24 hours. The volumetric ratio of polymer solution to ethanol is in the range of 20:1, more preferably in the range of 10:1 and even more preferably in the rage of 5:1.

Hydroxyl blockage or protection may be performed (step iv). Techniques known in the art can be applied. Preferably, the hydroxyls are blocked through acylation reaction using a compound such as ethanoyl chloride.

Residual levels of grafted anhydrides may also be present, preferably at a level below 0.05 mol/mol of polyacid.

The formation of grafted anhydrides may also be prevented through blockage of any free carboxylic acid groups prior to activation i.e. step (iv) taking place prior to step (ii).

Steps i) to iv) can be carried out in the presence of one or more solvents or catalysts, examples including dichloromethane (DCM), ethyl acetate (EtOAc) dimethylaminopyridine (DMAP), and triethylamine (TEA) or any combination thereof.

The purification step v) is carried out to ensure that any solvents and un-reacted products are removed from the pre-polymer made by step iii) and iv). This step can comprise filtration and/or water washing step. When this step v) comprises water washing step, conditions to allow a fast phase separation between organic and aqueous phase should be favored. For example, phase separation during water washings can be improved by the use of salts solubilized in the aqueous phase. Examples of salts include but are not limited to, sodium chloride, sodium bicarbonate. **In** alternative, the salts produced during the reaction can be removed through filtration using an organic solvent such as ethyl acetate, n-methyl tetrahydrofurane, tetrahydrofurane.

The purification step may also preferably be followed by one or more, more preferably all of the following steps including addition of free radical inhibitor, for example butylated hydroxytoluene (BHT), monomethylether-hydroquinone (MEHQ), phenylbutyl-nitrone (PBN), and/or photoinitiator, for example Irgacure 2595 or diphenyl-trimethyl-phosphine oxide (TPO), solvent evaporation and/or extraction, preferably through supercritical CO₂ to assure efficient solvent and impurities removal without interfering with the activation of the pre-polymer.

Preferably, the pre-polymer of the composition can be photopolymerised and/or photocured by light, preferably by UV light. The pre-polymer becomes a crosslinked polymeric material. The composition can additionally be cured by a Mitsunobu-type reaction, by redox-pair initiated polymerization for example benzoyl peroxide, N,N,-dimethyl-p-toluidine, ammonium persulfate, or tetramethylenediamine (TEMED), and by a Michael-type addition reaction using a bifunctional sulfhydryl compound. The term "printing" encompasses curing of the composition.

### Photo-Initiator

Preferably, the photo-initiator of the composition according to the present invention is diphenyl-trimethyl-phosphine oxide (TPO). Other examples of suitable photo-initiators include, but are not limited to: 2,4,6-trimethylbenzoyldiphenylphosphine oxide, 2-dimethoxy-2-phenyl-acetophenone, 2-hydroxy-1-[4-(hydroxyethoxy)phenyl]-2-methyl-1-propanone (Irgacure 2959), 1-hydroxycyclohexyl-1-phenyl ketone (Irgacure 184), 2-hydroxy-2-methyl- 1 -phenyl- 1-propanone (Darocur 1173), 2-benzyl-2-(dimehylamino)-1-[4-morpholinyl) phenyl] -1-butanone (Irgacure 369), methylbenzoylformate (Darocur MBF), oxy-phenyl-acetic acid-2-[2-oxo-2-phenyl-acetoxy-ethoxy]-ethyl ester (Irgacure 754), 2-methyl-1-[4-(methylthio)phenyl]-2-(4-morpholinyl)- 1-propanone (Irgacure 907), diphenyl(2,4,6-trimethylbenzoyl)-phosphine oxide (Darocur TPO), phosphine oxide, phenyl bis(2,4,6-trimethyl benzoyl) (Irgacure 819), and combinations thereof.

The photo-initiator may preferably have an absorption wavelength peaking between 365 to 420 nm.

According to another embodiment, said photo-initiator is sensitive to visible light (typically blue light or green light). Examples of photoinitiators sensitive to visible light include, but are not limited to eosin Y disodium salt, N-Vinyl-2-Pyrrolidone (NVP) and triethanolamine, and camphorquinone.

Photo-initiator Irgacure 2959 may be used which causes minimal cytotoxicity (cell death) over a broad range of mammalian cell types and species, however it is possible to reduce this risk by using non toxic amounts.

Preferably, the content of the photo-initiator is 0.1% to 1% w/w of the pre-polymer. Preferably, the concentration of the photo-initiator is in the range of 1000 to 10,000 ppm, preferably 4000 to 6000 ppm, most preferably 5000 to 6000 ppm. In some aspects the photo-initiator is TPO at a concentration of 5048 ppm. In this aspect a solvent may not be present in the composition. In some aspects the photo-initiator is TPO at a concentration of 5113 to 5213 ppm, optionally 5113 ppm, or optionally 5213 ppm. In this aspect a solvent may be present in the composition and the solvent may be 1-propanol, optionally at a concentration of 12%.

### Light Blocker

As used herein, the term "light blocker " includes any single compound or combination of compounds which absorbs or reflects light radiations, when incorporated into composition of the invention, such that transmission of light radiations is reduced. "Light blockers" are well known and commercially available.

According to a special embodiment, said "light blocker " absorbs or reflects light radiations selected in the group of blue radiations, infra-red radiations and/or UV radiations.

According to a special embodiment, said "light blocker " absorbs or reflects light radiations having a wavelength below 420 nm, preferably below 410 nm.

According to a special embodiment, said "light blocker " absorbs or reflects light radiations having a wavelength of 405 nanometers ± 7%.

According to a special embodiment, said "light blocker " absorbs or reflects light radiations having a wavelength below 500 nm, preferably below 480 nm.

According to a special embodiment, said "light blocker " absorbs or reflects light radiations having a wavelength above 650 nm.

According to a special embodiment, said "light blocker " absorbs or reflects all light radiations.

According to a preferred embodiment, said "light blocker " is an "UV blocker ". As used herein, the term "UV blocker " includes any single compound or combination of compounds which absorbs or reflects UV light, when incorporated into composition of the invention, such that transmission of UV light is reduced. Synonyms of "UV blocker " are "ultraviolet light absorber(s) or stabilizer(s)". "UV blockers" are well known and commercially available.

The presence of a UV blocker controls the depth of light penetration and scattering which enables a higher printed product resolution to be achieved. Preferably, the UV blocker has an absorption wavelength peaking between 350 to 500 nm. The UV blocker may also be screened to determine the optimal concentration before performing the 3D printing.

The preferred UV blocker according to the present invention is 2,5-Bis(5-tert-butyl-benzoxazol-2-yl)thiophene (BBOT). Other examples of suitable UV blockers include, but are not limited to: 2,2'-(2,5-thiophenediyl)bis(5-tert-butylbenzoxazole) (Mayzo OB+), 2-ethyl-9,10-dimethoxy anthracene, 1,4-Bis (2-methylstyryl) benzene, Oxybenzone, Dioxybenzone, 4-hydroxybenzophenone. Alternatively, nanoparticles or other light blocking particles may also be used instead of specific chemicals.

Preferably, the content of the UV blocker is 0.02% to 0.3% w/w of the pre-polymer. Preferably, the concentration of the UV blocker is in the range of 50 to 2500 ppm, more preferably of 500 to 2500 ppm,t preferably 1500 to 1800 ppm, most preferable 1600 to 1700 ppm. In some aspects the UV blocker is BBOT at a concentration of 1679 ppm. In this aspect a solvent may not be present in the composition. In some aspects the UV blocker is at a concentration of 1800 to 1823 ppm, optionally 1800 ppm, optionally, 1823 ppm, optionally 1809 ppm. In this aspect a solvent may be present in the composition and the solvent may be 1-propanol, optionally at a concentration of 12%.

### Solvent

The inventors have further realised that biocompatible polymers such as PGSA can exhibit increased viscosity at room temperature. Room temperature is generally the running temperature of many 3D printing platforms.

Therefore, according to one special embodiment, the composition further comprises at least one solvent. More particularly, this solvent is added to the composition when the printing step is conducted at room temperature (e.g. below 30°C, for example 25°C). According to preferred embodiment, said solvent has a boiling point of at least 90°C, more preferably at least 100°C, even more preferably at least 160°C. One preferred solvent is 1-propanol, which demonstrates good solubility and no swelling of the pre-polymer, particularly for the preferred PGSA while maintaining optimal viscosity at room temperature. Other preferred solvents are ethylene glycol, propylene glycol and N-Methyl-2-pyrrolidone which have higher boiling points (197.3°C, 188°C and 202°C, respectively). These prevent evaporation and maintain optimal viscosity at room temperature. The 3D products obtained using solvent in the composition have good structural properties such as high stability and resolution. Other examples of suitable solvents include, but are not limited to: glycerol, methanol, dimethyl sulfoxide, ethanol, nitromethane, dimethylformamide, dimethyl fumarate, isopropanol, acetonitrile, dioxane, pyridine, xylene and combinations thereof.

The solvent content can be up to 50% w/w of the pre-polymer, and preferably 5% to 20% w/w, in particular 12%.

**In** a preferred embodiment, at a printing temperature of 25°C the composition contains 25% ethanol w/w of the pre-polymer and has a viscosity of up to 220 cP.

**In** another preferred embodiment, at a printing temperature of 25°C the composition contains 15% 1-propanol w/w of the pre-polymer and has a viscosity of up to 1300 cP.

**In** a preferred embodiment, at a printing temperature of 25°C the composition contains 25% DMSO w/w of the prepolymer or polymer and has a viscosity of up to 1850 cP.

According to another embodiment, the composition of the invention does not contain any solvent. The Inventors have further shown that, when needed (e.g. in order to improve printability property), it is possible to decrease viscosity of the composition of the invention, especially when solvent is absent from the composition, by increasing the temperature during the printing step (for example to a temperature between about 30°C and about 40°C, more particularly about 35°C). Different combinations of solvents and printing temperatures may also be considered in the scope of the present invention.

According to one embodiment, the composition has a viscosity in the range of 10 to 30 000 cP, preferably, the composition has a viscosity in the range of 100 to 25000 cP, preferably 7000 to 14000 cP.

Preferably, the composition has a viscosity in the range of 100 to 6000 cP.

According to a preferred embodiment, the said viscosity of the composition is measured at 25°C.

Preferably, a printing temperature of 30°C is desired for a composition with a viscosity of up to 11000 cP, 40°C for a viscosity of up to 4300 cP, 50°C for a viscosity of up to 2200 cP, 65°C for a viscosity of up to 900 cP and 100°C for a viscosity of up to 200 cP.

Viscosity analysis can be performed using a Brookfield DV-II + Pro viscosimeter with a 2.2mL chamber and SC4-14 spindle. The speed during the analysis is varied from 5 to 80 rpm.

The composition may also comprise a radiopacity agent or contrast agent such as iodioxinal, ioxaglate, iohyexyl, iopromide and combinations thereof. This enables the implant to be viewed in situ using known imaging techniques.

### Other

The composition may further contain one or more pharmaceutical, therapeutic, prophylactic, and/or diagnostic agents. The agent may be a small molecule agent, for example having molecular weight less than 2000, 1500, 1000, 750, or 500 Da, a biomolecule, for example peptide, protein, enzyme, nucleic acid, polysaccharide, growth factors, cell adhesion sequences such as RGD sequences or integrins, extracellular matrix components, or combinations thereof. These may be agents which support cytological growth and survival. Exemplary classes of small molecule agents include, but are not limited to, anti-inflammatories, immunosupressants, neuroprotectants, anti-thrombotic agents, agents to support cytological growth and survival, analgesics, antimicrobial agents, and combinations thereof. Exemplary growth factors include, without limitation, neurotrophic factors, TGF-β, acidic fibroblast growth factor, basic fibroblast growth factor, epidermal growth factor, IGF-I and II, vascular endothelial-derived growth factor, bone morphogenetic proteins, platelet-derived growth factor, heparin-binding growth factor, hematopoetic growth factor, peptide growth factor, or nucleic acids and combinations thereof. The composition may further contain natural polymer and biopolymers, including extracellular matrix components. Exemplary extracellular matrix components include, but are not limited to, collagen, fibronectin, laminin, elastin and combinations thereof. Proteoglycans and glycosaminoglycans can also be covalently or non-covalently associate with the composition of the present invention.

Functional groups on the pre-polymer may be used to covalently attach one or more agents, such as small molecule agents and/or biomolecules. Alternatively, the one or more agents can be mixed with the composition of the invention before 3D printing such that it is physically entrapped within the 3D printed composition by curing the composition in the presence of the agent.

The composition may further include salt, proteins or glycans. This may be used as porogenic agents, for example, to confer porosity to the structure upon implantation. Examples may include glycans such as trehalose, glucose, hyaluronic acid, cyclodextrin, and the like.

### 3D Printing Method

The method of 3D printing the biomaterial of the present invention comprises:
(a) 3D printing the resin composition according to the invention, and
(b) washing the 3D printed composition with a solvent.

Step (a) of the 3D printing method can include the steps of :
(i) delivering a layer of the resin composition of the invention according to desired printing parameters
(ii) exposing the layer of the resin composition of the invention to light to cure the polymer of the resin and produce a solidified resin layer and
(iii) repeating step (i) and (ii) with each successive layer built upon the previous layer to obtain 3D printed composition.

Alternatively, the resin composition of the invention can further be used in continuous generative process for producing a 3D object (see for example methods disclosed in WO2014126837 or US7892474).

Preferably, the 3D printing method is a digital light processing (DLP) method. DLP typically requires a vat of photoreactive material which is selectively exposed to light in order to create solid layers which are stacked upon one another to form a 3D structure. Other examples of 3D-printing methods include, but are not limited to, laser based stereolithography (SLA), continuous liquid interface production (CLIP), ink-jet printing, projection stereolithography and combinations thereof.

The 3D printer system is preferably an Asiga, 3D Systems or Envisiontech. Other examples of platforms include, but are not limited to, Ember platform from Autodesk. Preferably, a vat with a PDMS or a Teflon or a fluorinated polymer membrane is utilised.

Methods of 3D printing the resin require printing parameters, considering the chemical composition of the resin, to be defined before the final structure is generated. A digital representation of the biomaterial can be generated used Computer Aided Design (CAD) modelling. These printing parameters can be customised for an individual bespoke structure, polymer formulation and/or targeted mechanical properties, or a library of printing parameters can be created, for example, to meet the requirements for an implant for a specific medical application. This encourages high fidelity between printed structures. Accordingly, other aspects of the method such as washing, vacuuming or heating may be adjusted for a specific implant for a specific medical application.

Preferably, step (a) is carried out at a temperature in the range of room temperature to 110°C, most preferably in the range of 25°C to 95°C, more preferably of 35°C to 95°C, even more preferably of 30°C to 60°C. Preferably the pressure is atmospheric.

Step (a) may be carried out at room temperature in the presence of at least one solvent. Examples of suitable solvents include, but are not limited to: glycerol, ethylene glycol, 1-propanol, propylene glycol, methanol, dimethyl sulfoxide, ethanol, nitromethane, dimethylformamide, dimethyl fumarate, isopropanol, acetonitrile, dioxane, N-Methyl-2-pyrrolidone (NMP), preferably 5 to 20% of NMP, pyridine, xylene and combinations thereof. According to preferred embodiment, the solvent is selected from any of 1-propanol, ethylene glycol, propylene glycol and N-Methyl-2-pyrrolidone.

Step (a) may be carried out in absence of any solvent.

Preferably, step (a) is carried out at a light wavelength of between 365 to 415nm, most preferably 405nm. Preferably, the power source is in the range of 20 to 100 mW/cm², most preferably 100 mW/cm².

Preferably, the solvent in step (b) is mixed with the 3D printed composition at temperature from 4°C to 40°C at a preferred mass ratio. Alternatively, the solvent in step (b) is mixed with the 3D printed composition at room temperature to 40°C at a preferred mass ratio. The solvent may be mixed in a closed vial via agitation and/or sonication. Alternatively, the solvent is mixed with the composition prior to 3D printing.

Preferably, the solvent in step (b) is selected from the class of oxygenated organic solvents such as alcohols, glycol ethers, methyl acetate, ethyl acetate, ketones, esters, and glycol ether/esters. Examples of suitable solvents include, but are not limited to: isopropyl alcohol, acetone, ethyl acetate, diethyl ether, tetrahydrofuran, dichloromethane, N-Methyl-2-pyrrolidone, dimethyl sulfoxide. According to preferred embodiment, the solvent in step (b) is ethanol, which has low toxicity and is easy to eliminate.

Step (b) may be undertaken to remove any uncured resin from the 3D printed composition, to remove excess UV blocker and/or to remove excess photo-initator. The printed biomaterial is immersed in the solvent, preferably at 1ml of solvent per 10mg of printed biomaterial. Preferably, the solvent is vortexed, sonicated, and/or dynamic stirred for a period of 18 to 24 hours. Preferably, the solvent is exchanged between 3 to 6 times during step (b).

Step (b) may result in the removal of up to 96% extraction of the UV blocker. Step (b) may result in the removal of up to 45% of the photo-initiator. This results in a 3D printed biomaterial (e.g. biocompatible implant) characterized by low toxicity (including cellular toxicity).

The method may further comprise the step of (c) vacuuming the washed 3D printed composition. Preferably, the vaccum is operated at <50 mbar for at least 1 hour. The present inventors have realized that if this step is not performed cracking of samples might surprisingly often be observed. This step therefore greatly improves structural integrity and resolution.

The method may further comprise the step of (d) post-curing the 3D printed composition through exposure to light, preferably to UV or blue light.

The method may further comprise the step of (e) heating the washed 3D printed composition. This step may result in the removal of excess solvent. Step (e) may also result in thermal curing of the printed composition, which enables reinforcement of mechanical properties and sample shrinkage especially if the composition is printed in the presence of a solvent.

Preferably, the heating of step (e) occurs in a ramp fashion to prevent thermal shock and potential cracking.

Preferably, step (e) is carried out at a temperature in the range of 120°C to 150 °C for 1 to 5 days, most preferably at 120°C to 140°C for 1 to 4 days.

Preferably, the 3D printed composition comprises printed layers of a plane resolution of 25µm to 50µm and an axial resolution of between 1 µm to 100µm, preferably between 10µm to 100µm, most preferably 50µm, even more preferably 10 µm.

Preferably, the final printed composition has a solvent presence of <150 ppm, preferably <134 ppm (limit of quantitation). This results in a printed composition with lower toxicity.

Preferably, the final printed composition exhibits less than 30% volume shrinkage, more preferably less than 20% volume shrinkage and even more preferably less than 10% volume shrinkage throughout the 3D printing process, and optionally post-processing. Shrinkage of the printed composition may be utilized in order to reduce the overall size of the printed structure whilst maintaining the same relative dimensions.

### Biomaterials

Also provided herein is a biomaterial, such as a biocompatible implant or other medical device, including non-implantable functions, obtainable by the method according to the present invention.

The biomaterial of the invention is preferably sufficiently elastic to resist movement of the underlying tissue, for example contractions of the heart and blood vessels. The biomaterial such as a biocompatible implant is preferably biodegradable and biocompatible, causing minimal *in vivo* inflammatory response. The biomaterial is preferably elastomeric.

Biodegradability can be evaluated in vitro, such as in phosphate buffered saline (PBS) or in acidic or alkaline conditions. Biodegradability can also be evaluated in vivo, such as in an animal, for example mice, rats, dogs, pigs or humans. The rate of degradation can be evaluated by measuring the loss of mass and/or thickness of the biocompatible implant over time in vitro or in vivo.

The biomaterial may contain and/or may be coated with one or more pharmaceutical, therapeutic, prophylactic, and/or diagnostic agents. The agent may be a small molecule agent, for example having molecular weight less than 2000, 1500, 1000, 750, or 500 Da, a biomolecule, for example peptide, protein, enzyme, nucleic acid, polysaccharide, growth factors, cell adhesion sequences such as RGD sequences or integrins, extracellular matrix components, or combinations thereof. Exemplary classes of small molecule agents include, but are not limited to, anti-inflammatories, anti-thrombotic agents, agents to support cytological growth and survival, analgesics, antimicrobial agents, and combinations thereof. Exemplary growth factors include, without limitation, neurotrophic factors, TGF-β, acidic fibroblast growth factor, basic fibroblast growth factor, epidermal growth factor, IGF-I and II, vascular endothelial-derived growth factor, bone morphogenetic proteins, platelet-derived growth factor, heparin-binding growth factor, hematopoetic growth factor, peptide growth factor, or nucleic acids and combinations thereof. Exemplary extracellular matrix components include, but are not limited to, collagen, fibronectin, laminin, elastin and combinations thereof.

The use of a biodegradable material allows the elution of agents from the implant over time.

The biomaterial, such as a biocompatible implant, may be custom printed according to the subject who is receiving the implant. The biomaterial may be custom printed according to the intended application, for example, increased length and flexibility, e.g. for a nerve conduit. The biomaterial may be custom printed according to the duration of time it is intended to remain in situ and/or perform a particular function.

The implant may comprise complex microstructures such as projected micro-channels, ridges, positive and/or negative grooves and/or projections.

Preferably, the biomaterial, such as a biocompatible implant, obtainable by the method has one or more of the following properties: full recovery after 60% compression (rebound test), full recovery after a 90° bend (3-point bend test) and/or break at elongation between 20 to 50%.

Preferably, the biomaterial obtainable by the method is a biocompatible implant.

Preferably, the biocompatible implant obtainable by the method is a nerve conduit.

Preferably, the implant has a wall thickness of 50 to 500µm.

The biomaterial may be for use inside and outside the body, and for human or veterinary use. The implant may be for research or educational use.

Also provided herein is a method of repairing nervous tissue, the method comprising applying the biomaterial, preferably a biocompatible implant obtainable by the method according to the present invention to nervous tissue.

The biomaterial may also contain one or more types of cells, such as connective tissue cells, organ cells, muscle cells, nerve cells, and combinations thereof. Optionally, the material is seeded with one or more of tenocytes, fibroblasts, ligament cells, endothelial cells, lung cells, epithelial cells, smooth muscle cells, cardiac muscle cells, skeletal muscle cells, islet cells, nerve cells, hepatocytes, kidney cells, bladder cells, urothelial cells, chondrocytes, and bone-forming cells. The combination of cells with the material may be used to support tissue repair and regeneration.

The biomaterial can be used as a tissue support or scaffold to serve a supportive function. Such implants may exert functions such as holding or bridging two tissues together or positioning the tissue in a specific position inside or outside the body.

The implant can be coated with cells and/or tissue, for example a nerve graft in order to be implanted adjacent to or contacting a damaged nerve. The cells and/or tissue may be cultured on the implant or/and in the lumen of the implant prior to in vivo situation according to generally known cell culture methods.

The implant may also be custom printed according to a desired porosity, elasticity or shape for optimal cell culture and support. Porosity may be at meso-scale (<1µm).

The invention further concerns the use of the composition according to the invention as bioink. In this case it can further comprise coloring agent.

The present invention will now be illustrated, but in no way limited, by reference to the following examples.

### EXAMPLES

In the following, PGSA was manufactured according to WO 2016/202984.

### Example 1 - Printing with and without solvent

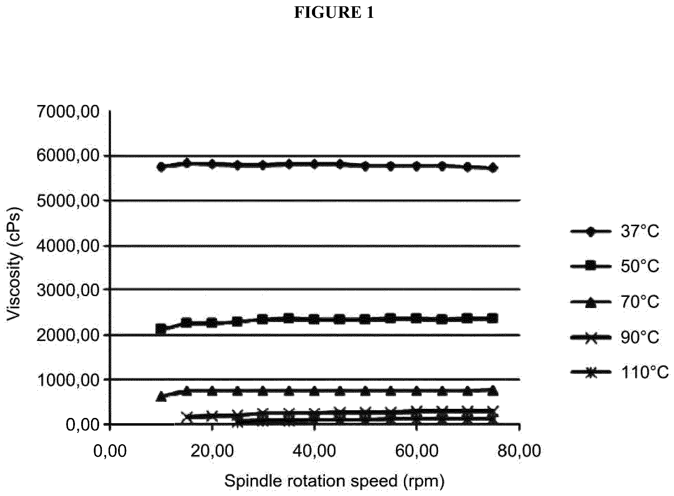

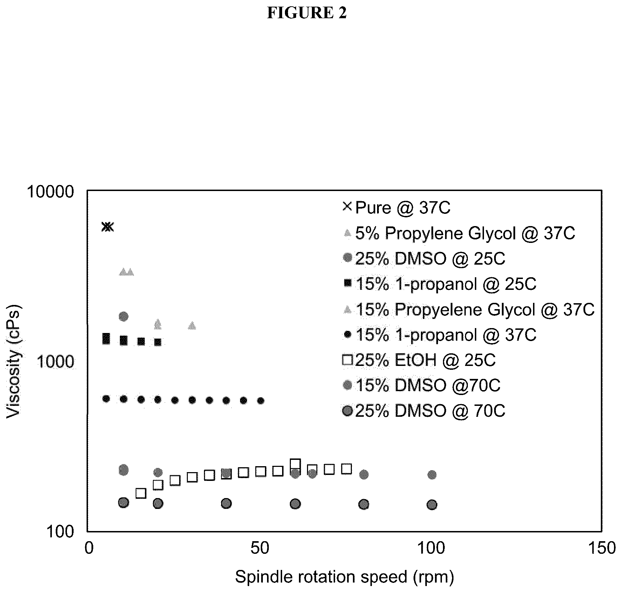

Two PGSA resin compositions were developed to enable printing of PGSA resin. The first was used for printing at high temperatures (e.g. 100-110°C) in the absence of solvent. Viscosities at different temperatures are shown in Figure 1. The second composition was developed for printing at room temperature in the presence of solvent. The viscosities of PGSA resin compositions in the presence of various solvents are shown in Figure 2. All compositions comprised (in the range of 5000 ppm TPO and 1600 ppm of BBOT - see below). The compositions were 3D printed at atmospheric pressure using a commercially available Autodesk Ember 3D printer, equipped with a 405 nm LED, power source 90mW/cm², and using the Digital Light Processing (DLP) method. The printed parts were then removed from the printer and washed in ethanol to remove uncured resin, BBOT and TPO. The 3D constructs were immersed in extraction solvent (1mL/10mg of construct), diffusion aided by vortexing or dynamic stirring for a period of 18 to 24 hours. The solvent was exchanged 3 to 6 times.

The table below shows the results of solvent extraction using different solvents:

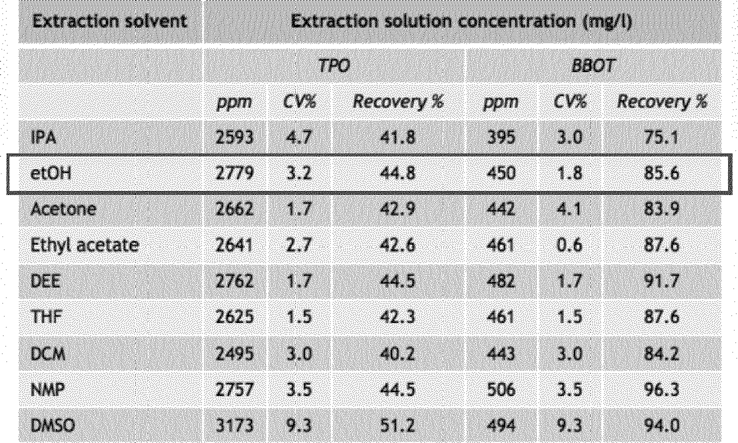

Ethanol was then removed in a step wise approach. The first step was solvent evaporation by vacuum (< 50 mbar) for at least 1 hour. It was observed that if this step is not performed, there was cracking of samples. The second step was heating at 140°C for 4 days which eliminated remaining solvent. Initial heat ramps prevented thermal shock and potential cracking and enabled reinforcement of mechanical properties.

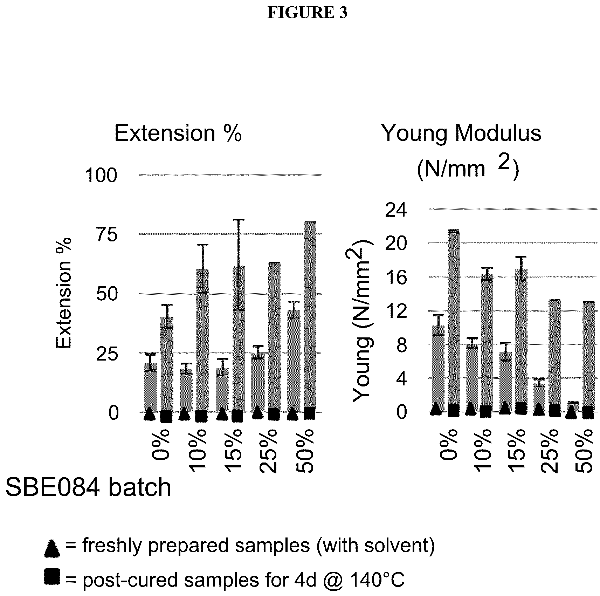

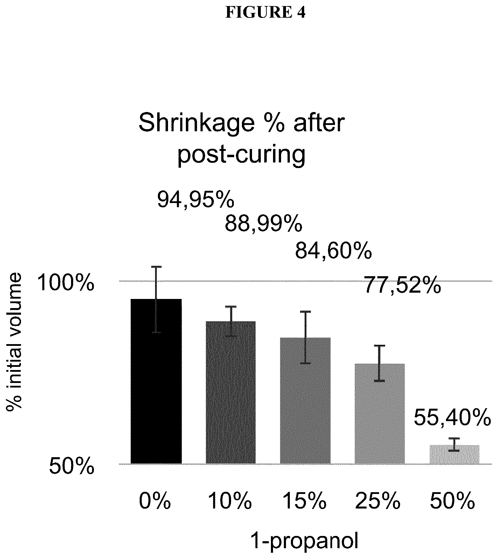

The post processing conditions enabled improving the mechanical (elasticity, modulus) of the final conduits. If printed in the presence of tested solvent 1-propanol, some shrinkage may occur. The results can be seen in Figures 3 and 4.

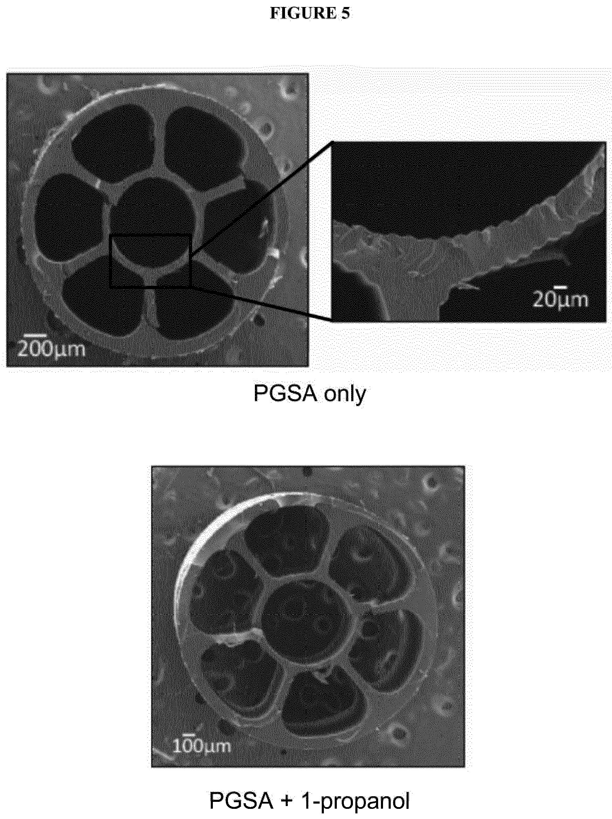

Figure 5 shows the 3D printed products using PGSA alone (with 5048ppm of TPO, and 1679ppm of BBOT), and PGSA with 1-propanol (with 5113 ppm of TPO and 1809 ppm of BBOT) which are suitable as implantable nerve conduits. The conduits were washed in ethanol prior to Scanning Electron Microscopy (SEM) imaging.

### Example 2 - Printing in absence of solvent

A PGSA composition comprising 5200 ppm TPO and 1800 ppm BBOT without any solvent was printed using ASIGA PICO2 HD DLP printer at atmospheric pressure equipped with a 405nm LED, power source 70mW/cm², and using the Digital Light Processing (DLP) method.

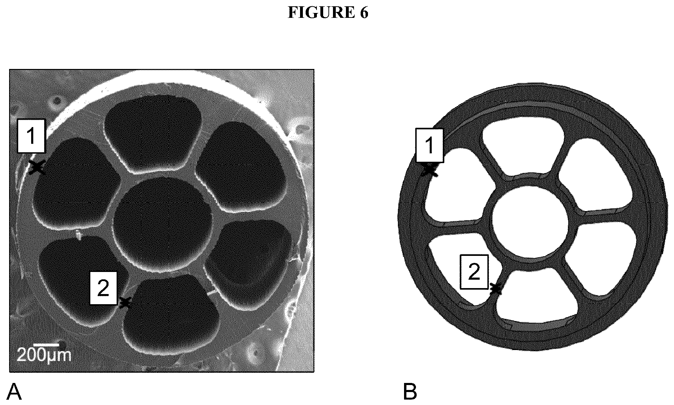

The temperature of the printer chamber was set to be 50°C during the printing. The 3D printed parts (Figure 6) were then removed from the printer and washed in ethanol to remove uncured resin, BBOT and TPO.

Scanning Electron Microscopy images of the obtained printed parts were taken. The wall thickness of the object sent to print was of 100µm with a wall thickness of the printed part of 131.88±10.71µm (see "1" in Figure 6). The struts of the object sent to print was of 100µm with struts of the printed part of 124.67±14.62µm (see "2" Figure 6).

### Example 3- Printing in absence of solvent

A PGSA composition comprising 5213 ppm TPO and 1823 ppm BBOT without any solvent was printed using 3D Systems DLP printer.

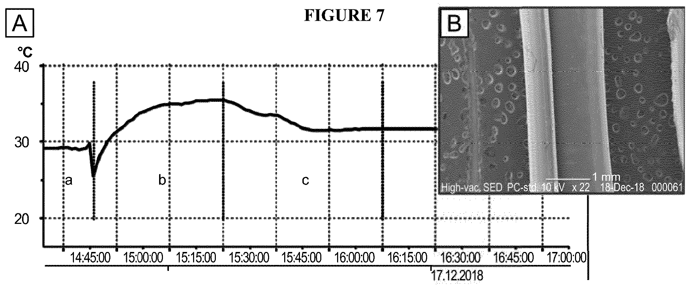

The initial temperature of the composition in the vat was measured to be 29°C (see Figure 7A section (a)). The viscosity value at this temperature for this composition was 13250 cP.

During the 3D printing process, the composition temperature initially rose up to 35°C (Figure 7A section (b)), due to long exposure times set to print the base-layer. At this temperature the composition viscosity was estimated to be 7859 cP.

In Figure 7A section (c), the temperature profile during the printing is reported: the temperature decreases since exposure times are shorter than the ones set for the base layer.

Figure 7B shows a nerve wrap produced according to the said conditions. More particularly, it shows that despite the high viscosity of the PGSA composition at low temperature, the print outcomes were successful.

### Example 4- Impact of UV blocker on resin printability

Two PGSA resin compositions were tested to validate the usability of PGSA as a 3D printing resin. The first formulation was PGSA composition in absence of BBOT while the second formulation was supplemented with 1800 ppm BBOT. Both compositions comprised 5000 ppm TPO.

The formulations were 3D printed at atmospheric pressure using a commercially available ASIGA PICO2 HD printer, equipped with a 405nm LED, power source 70mW/cm², and using the Digital Light Processing (DLP) method. The printed parts were then removed from the printer and washed in ethanol to remove uncured resin then dried in vacuum.

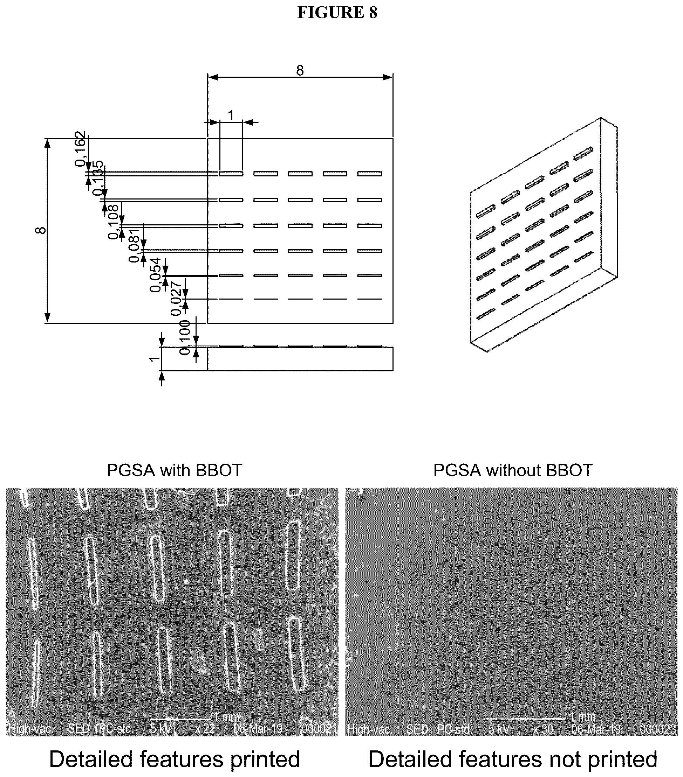

The 3D constructs comprise a 8mm x 8mm base with a thickness of 1mm and a series of small detailed features of 100µm in height as show in Figure 8 (drawing). The printed 3D constructs were placed inside a scanning electron microscope (SEM) to observe the presence of the detailed features on the base, as shown in Figure 8 (SEM pictures).

Figure 8 shows the presence of the detailed features on the 3D construct printed with the first formulation and the absence of the detailed features on the 3D construct printed with the second formulation. The base successfully printed for the two formulations.

### Example 5- Elasticity of 3D printed Biomaterials

A PGSA resin composition comprising 5213 ppm TPO and 1823 ppm BBOT was printed without any additional solvent in a 3D Systems DLP printer.

The post-processing consisted of successive ethanol washes, drying for 30 min under vacuum prior to thermal curing at 140°C for 4 days.

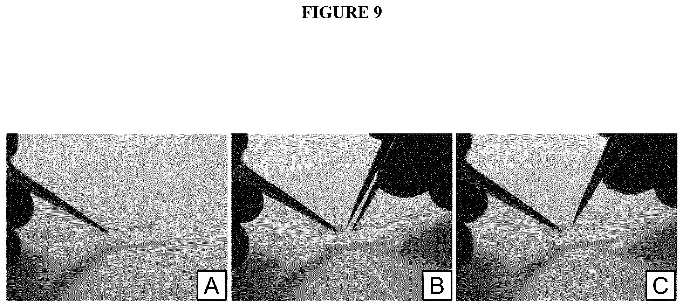

As seen in Figure 9B, the wrap can be easily opened with surgical tweezers and it recovers its original shape when tweezers are removed (Figure 9C).

### Example 6- Biocompatibility of 3D printed Biomaterials

A PGSA resin composition comprising 5113 ppm TPO, 1809 ppm BBOT and 12% 1-Propanol to reduce the resin viscosity was printed in a Ember DLP printer. The parts produced where post-processed according to the protocol described above. Briefly, after printing samples were washed by successive ethanol baths (3) under agitation for 18h. Samples were then dried under vacuum for 1h prior to thermal curing for 4 days at 140°C.

Printed parts (conduits) were then implantated at the level of the sciatic nerve in a rat model for 4 months. Tissue samples were fixed in formalin 10% before embedding, and cut in paraffin. Hematoxillin and Eosin staining was preformed to assess tissue respone to the material. Minimum to mild inflammatory response was observed at the level of the nerve tissue in response to the 3D printed material illustrating the biocompatibility of the 3D printed biomaterial.

### Example 7- Biodegradability of 3D printed Biomaterials

A PGSA resin composition comprising 5113 ppm TPO, 1809 ppm BBOT and 12% 1-Propanol to reduce the resin viscosity was printed in a Ember DLP printer. The parts produced where post-processed according to the protocol described above. Briefly, samples after printing were washed by successive ethanol baths (3) under agitation for 18h. Samples were then dried under vacuum for 1h prior to thermal curing for 4 days at 140°C.

The biodegradation of the samples was evaluated *in vitro* through exposure to 0.05M of NaOH aqueous solution. The shape of the parts was monitored for 7 days. This experiment has shown that biodegradation is observed over time.

### Also included herein are the following clauses:

1. 3D printing resin composition for a biomaterial, wherein the composition comprises:
   (i) a pre-polymer comprising a polymeric unit of the general formula (-A-B-)ₙ, wherein A represents a substituted or un-substituted ester, B represents a substituted or un-substituted acid ester comprising at least two acid ester functionalities, and n represents an integer greater than 1 ,
   (ii) at least one photo-initiator, and
   (iii) at least one light blocker.
2. The composition according to clause 1, wherein the pre-polymer has the following formula (I): 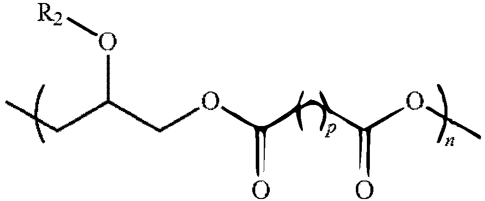 wherein n and p each independently represent an integer equal or greater than 1, and wherein R₂ in each individual unit represents hydrogen or a polymer chain or -C(=O)-CR₃=CR₄R₅, wherein R₃, R₄, R₅ are independently from one another, selected in the group consisting of H, alkyl such as methyl or ethyl, aryl such as phenyl, substituted alkyl, substituted aryl, carboxylic acid, ester , amide, amine, urethane , ether, and carbonyl.
3. The composition according to clause 1, wherein the pre-polymer has the following formula : 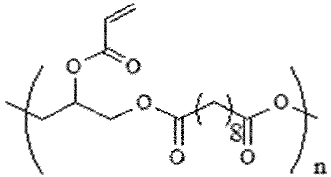 wherein n represents an integer equal or greater than 1.
4. The composition according to clause 1 or 3, wherein the photo-initiator is 2, 4, 6-trimethylbenzoyldiphenylphosphine oxide (TPO).
5. The composition according to any preceding clause, wherein the concentration of the photo-initiator is in the range of 1000 to 10,000 ppm.
6. The composition according to any preceding clause, wherein the light blocker is a UV blocker, preferably 2,5-Bis(5-tert-butyl-benzoxazol-2-yl)thiophene (BBOT).
7. The composition according to any preceding clause, wherein the concentration of the light blocker is in the range of 1200 to 2000 ppm.
8. The composition according to any preceding clause, wherein the composition further comprises a solvent.
9. The composition according to clause 8, wherein the solvent is any of 1-propanol, ethylene glycol, propylene glycol or N-Methyl-2-pyrrolidone .
10. The composition according to any preceding clause, wherein the composition has a viscosity in the range of 10 to 30 000 cP at 25°C.
11. A method of 3D printing a biomaterial, wherein the method comprises:
   (a) 3D printing the resin composition according to any preceding clause, and
   (b) washing the 3D printed composition with a solvent.
12. The method according to clause 11, wherein the solvent is ethanol.
13. The method according to clause 1 or 12, wherein step (a) is carried out at a temperature in the range of 35°C to 95°C.
14. The method according to according to clause 1 or 12, wherein step (a) is carried out at room temperature in the presence of a solvent, optionally wherein the solvent is any of 1-propanol, ethylene glycol, propylene glycol or N-Methyl-2-pyrrolidone .
15. The method according to any of clauses 11 to 14, further comprising: (c) vacuuming the washed 3D printed composition.
16. The method according to any of clauses 11 to 15, further comprising:
   (d) post-curing the parts using light and/or
   (e) heating the washed 3D printed composition
17. The method according to clause 16, wherein (e) is carried out at a temperature in the range of 130 to 150 °C for 3 to 5 days.
18. A biomaterial obtainable by the method according to any of clauses 11 to 17.
19. A biomaterial according to clause 18 with one or more of the following properties:
   full recovery after 60% compression (rebound test) and/or full recovery after a 90° bend (3-point bend test).
20. Use of the biomaterial according to clause 18 or 19 as a nerve conduit.
21. A method of repairing nervous tissue, the method comprising applying the biomaterial according to any of clauses 18 or 19 to a nervous tissue.
22. The biomaterial according to any preceding clause wherein the biomaterial is a biocompatible implant.
23. Use of the biomaterial according to any preceding clause as bioink

## Claims

1. 3D printing resin composition for a biomaterial, wherein the composition comprises:
(i) a pre-polymer comprising a polymeric unit of the general formula (-A-B-)ₙ, wherein A represents a substituted or un-substituted ester, B represents a substituted or un-substituted acid ester comprising at least two acid ester functionalities, and n represents an integer greater than 1,
(ii) at least one photo-initiator, wherein the photo-initiator is 2, 4, 6-trimethylbenzoyldiphenylphosphine oxide (TPO), and
(iii) at least one light blocker, wherein the light blocker is 2,5-Bis(5-tert-butyl-benzoxazol-2-yl)thiophene (BBOT).

2. The composition according to claim 1, wherein the pre-polymer has the following formula (I): 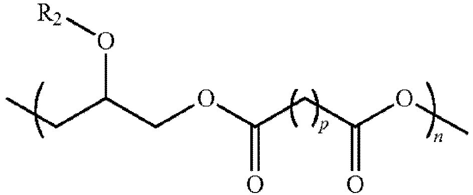 wherein n and p each independently represent an integer equal or greater than 1, and wherein R₂ in each individual unit represents hydrogen or a polymer chain or -C(=O)-CR₃=CR₄R₅, wherein R₃, R₄, R₅ are independently from one another, selected in the group consisting of H, alkyl such as methyl or ethyl, aryl such as phenyl, substituted alkyl, substituted aryl, carboxylic acid, ester, amide, amine, urethane, ether, and carbonyl.

3. The composition according to claim 1, wherein the pre-polymer has the following formula: 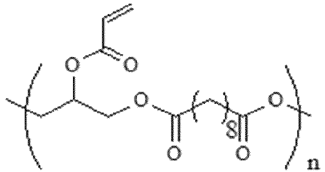 wherein n represents an integer equal or greater than 1.

4. The composition according to any preceding claim, wherein the concentration of the photo-initiator is in the range of 1000 to 10,000 ppm.

5. The composition according to claim 4, wherein the concentration of the photo-initiator is in the range of 4000 to 6000 ppm, preferably 5000 to 6000 ppm.

6. The composition according to any preceding claim, wherein the concentration of the light blocker is in the range of 1200 to 2000 ppm, preferably 1500 to 1800ppm, or more preferably 1600 to 1700ppm.

7. The composition according to any preceding claim, wherein the composition further comprises a free radical inhibitor, preferably the free radical inhibitor is any of butylated hydroxytoluene (BHT), monomethylether-hydroquinone (MEHQ) or phenylbutyl-nitrone (PBN).

8. The composition according to any preceding claim, wherein the composition further comprises a solvent, preferably wherein the solvent is any of 1-propanol, ethylene glycol, propylene glycol or N-Methyl-2-pyrrolidone.

9. The composition according to any preceding claim, wherein the composition has a viscosity in the range of 10 to 30 000 cP at 25°C.

10. A method of 3D printing a biomaterial, wherein the method comprises:
(a) 3D printing the resin composition according to any preceding claim, and
(b) washing the 3D printed composition with a solvent, preferably wherein the solvent is ethanol.

11. The method according to claim 10, wherein step (a) is carried out at a temperature in the range of 35°C to 95°C; or wherein step (a) is carried out at room temperature in the presence of a solvent, optionally wherein the solvent is any of 1-propanol, ethylene glycol, propylene glycol or N-Methyl-2-pyrrolidone.

12. The method according to claim 10 or 11, further comprising:
(c) vacuuming the washed 3D printed composition; and/or
(d) post-curing the parts using light; and/or
(e) heating the washed 3D printed composition.

13. The method according to claim 12, wherein (e) is carried out at a temperature in the range of 130 to 150 °C for 3 to 5 days.

14. A biomaterial obtainable by the method according to any of claims 10 to 13, optionally wherein the biomaterial is a biocompatible implant, preferably the biomaterial is a nerve conduit, more preferably a nerve wrap.

15. A biomaterial according to claim 14 for use in a method comprising applying the biomaterial to a nervous tissue.
